# EUROPEAN PATENT SPECIFICATION

(11) **EP 2 011 781 B1**
(45) Date of publication and mention of the grant of the patent: **05.03.2014**
(21) Application number: 07738607.6
(22) Date of filing: 14.03.2007
(51) Int. Cl.: C07C 45/29, C07C 45/28, C07C 45/58, C07C 49/743, C07C 49/753, C07C 67/313, C07C 69/145, C07C 401/00, C07D 301/14, C07F 7/18, C07B 53/00

(54) **PROCESS FOR PRODUCTION OF INDENE DERIVATIVE, AND INTERMEDIATE FOR PRODUCTION OF THE DERIVATIVE**
VERFAHREN ZUR HERSTELLUNG EINES INDENDERIVATS, UND ZWISCHENPRODUKT FÜR DIE HERSTELLUNG DES DERIVATS
PROCEDE DE SYNTHESE D'UN DERIVE D'INDENE ET INTERMEDIAIRE DE SYNTHESE DUDIT DERIVE

(30) Priority: 15.03.2006 JP 2006070248
(43) Date of publication of application: 07.01.2009
(73) Proprietor: MicroBiopharm Japan Co., Ltd., Chuo-ku Tokyo 103-0023 (JP)
(72) Inventor: TOYODA, Asako, Shizuoka, 4380078 (JP); NAGAI, Hazuki, Shizuoka, 4380078 (JP)
(74) Representative: Patentanwälte Ruff, Wilhelm, Beier, Dauster & Partner
(86) International application number: PCT/JP2007/055137
(87) International publication number: WO 2007/105773

(56) References cited:
- WO-A1-95/19963
- JP-A- 58 216 179
- JP-A- 59 176 250
- HANEKAMP J C ET AL: "25-hydroxydihydrotachysterol2 an innovative synthesis of a key metabolite of dihydrotachysterol2", TETRAHEDRON, ELSEVIER SCIENCE PUBLISHERS, AMSTERDAM, NL, vol. 48, no. 42, 1 January 1992 (1992-01-01), pages 9283-9294, XP026636787, ISSN: 0040-4020, DOI: 10.1016/S0040-4020(01)85618-0 [retrieved on 1992-01-01]
- STEPHEN R. WILSON ET AL: "An Intramolecular Diels-Alder Approach to the Cis Ring Fused Isomer of the 25-Hydroxy Vitamin D2 Grundmann Ketone", J. ORG. CHEM, vol. 57, 1992, pages 4380-4385, XP002684100,
- SARDINA F.J. ET AL.: 'Studies on the Synthesis of Side-Chain Hydroxylated Metabolites of Vitamin D. 2. Stereocontrolled Synthesis of 25-Hydroxyvitamin D2' J. ORG. CHEM. vol. 51, 1986, pages 1264 - 1269, XP003017769
- BLAKEMORE P.R. ET AL.: 'The Modified Julia Olefination in Vitamin D2 Synthesis' SYNTHESIS no. 7, 1999, pages 1209 - 1215, XP003017770
- SICINSKI R.R. ET AL.: 'Synthesis and Biological Activity of 1alpha, 25-Dihydroxy-18-norvitamin D3 and 1alpha, 25-Dihydroxy-18,19-dinorvitamin D3' J. MED. CHEM. vol. 39, 1996, pages 4497 - 4506, XP001026100

## Description

### [Technical Field]

The present invention relates to a method for preparing indene derivatives that are utilizable as intermediates in the synthesis of the vitamin D₂ derivative paricalcitol, which is useful as pharmaceutical, and to intermediates in the preparing thereof.

### Cross-reference to Related Applications

The present application claims priority under Japanese Patent Application 2006-70248, filed on March 15, 2006.

### [Background Art]

Paricalcitol, a vitamin D₂ derivative denoted by formula (A), has been discovered to exhibit differentiation-inducing activity on malignant cells (see Patent Reference 1), and is widely employed to treat hyperthyroidism in patients suffering from chronic renal failure. Known methods of preparing vitamin D derivatives of the 19-nor-type, including paricalcitol, include a method employing a starting material in the form of 25-hydroxyvitamin D derivatives (see Japanese Translation of PCT International Application Heisei No. 3-505330 or the English language family member thereof, WO 90/10620). Methods for obtaining such compounds by reacting an indene derivative and a phosphine oxide derivative (Japanese Unexamined Patent Publication (KOKAI) Heisei No. 5-221960 and the English language family members thereof, U.S. Patents 5,281,731 and 5,391,755,) are known to be more efficient methods.

Numerous methods are known for preparing the indene derivatives employed in the above-cited methods (see U.S. Patent 4,804,502; J. Org. Chem. 51, 3098 (1986); J. Org. Chem. 51, 1264 (1986); J. Chem. Soc. Perkin Trans. 1, 834 (1978); J. Org. Chem. 48, 1414 (1983); and J. Org. Chem. 51, 1269 (1986); Sicinski et al., J. Med. Chem 1996, 39, 4497-4506 disclose also the synthesis of 1α,25-Dihydroxy-18-norvitamine D₃ and 1α,25-Dihydroxy-18,19-dinorvitamine D₃ ). However, all methods are unsatisfactory. These are all methods in which the double bonds at positions 7 and 8 and positions 22 and 23 of vitamin D₂ are severed by a chemical method such as ozone degradation, after which a 25-hydroxylated vitamin D₂ side chain (position 23 and beyond) that has been separately synthesized in multiple steps is introduced onto the indene derivative with severed side chains.And other methods in which an extremely large number of steps are employed to hydroxylate position 25 of an indene derivative with severed side chains.

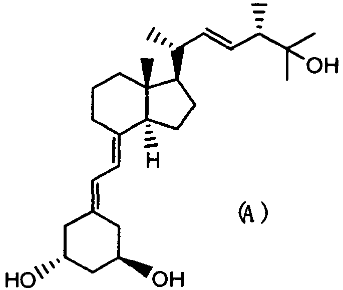

The present invention provides a novel method for preparing the indene derivatives denoted by formula (III) wherein in formula (III), R₁ denotes a hydrogen atom or a protective group for a hydroxyl group and formula (III)', an implementation form thereof, that are utilizable particularly as intermediates in the synthesis of paricalcitol; as well as intermediates in the preparing thereof.

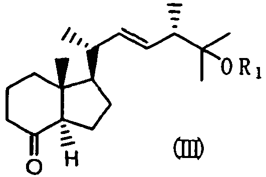

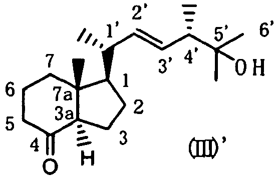

### [Disclosure of Invention]

The present inventors conducted extensive research into achieving the above-stated object, resulting in the discovery that the indene derivative denoted by formula (III) above could be efficiently prepared by subjecting 25-hydroxyvitamin D₂ to a two-steps oxidation reaction in a suitable solvent. The present invention was devised based on this knowledge.

Accordingly, the present invention provides inventions [1] to [22] below.
[1] A method for preparing an indene derivative denoted by formula (III) 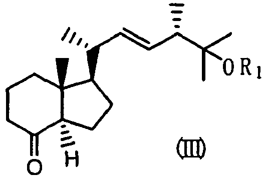 wherein in formula (III), R₁ denotes a hydrogen atom or a protective group for a hydroxyl group, characterized by comprising oxidizing a vitamin D₂ derivative denoted by formula (V) 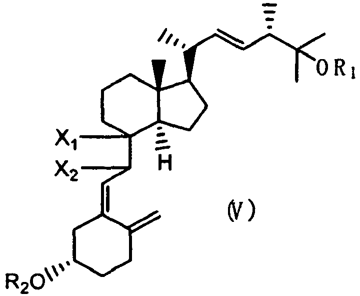 wherein in formula (V), both X₁ and X₂ denote hydroxyl groups or jointly form an epoxy group, and each of R₁ and R₂ independently denotes a hydrogen atom or a protective group for a hydroxyl group.
[2] The preparing method in accordance with [1], wherein the oxidation of the vitamin D₂ derivative denoted by formula (V) is conducted in solvent with an oxidizing agent.
[3] The preparing method in accordance with [1] or [2], wherein the protective groups for hydroxyl groups denoted by R₁ and R₂ are silyl groups, alkoxymethyl groups, aralkyloxymethyl groups, or acyl groups.
[4] The preparing method in accordance with [1] or [2], wherein the protective groups for hydroxyl groups denoted by R₁ and R₂ are trimethylsilyl groups, triethylsilyl groups, methoxymethyl groups, benzyloxymethyl groups, or acetyl groups.
[5] The preparing method in accordance with [1] or [2], wherein the vitamin D₂ derivative denoted by formula (V) is 7,8,25-trihydroxyvitamin D₂ denoted by formula (II) 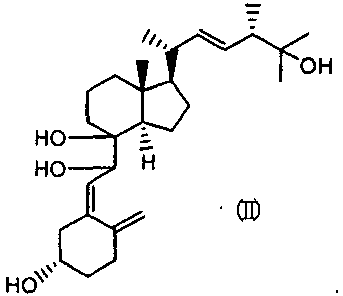
[6] The preparing method in accordance with any one of [1] to [5], wherein the vitamin D₂ derivative denoted by formula (V) is prepared by oxidizing the vitamin D₂ derivative denoted by formula (IV) 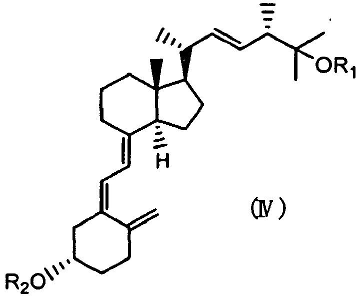 wherein in formula (IV), both R₁ and R₂ are defined as in formula (V).
[7] The preparing method in accordance with [6], wherein the oxidation of the vitamin D₂ derivative denoted by formula (IV) is conducted in solvent with an oxidizing agent.
[8] The preparing method in accordance with [5] wherein the 7,8,25-trihydroxyvitamin D₂ denoted by formula (II) is prepared by oxidizing the 25-hydroxyvitamin D₂ denoted by formula (I) 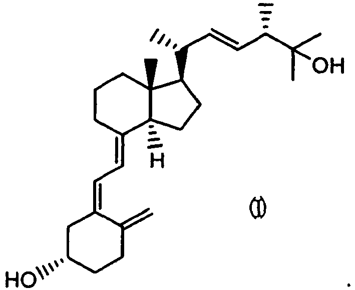
[9] A method for preparing a vitamin D₂ derivative denoted by formula (V) 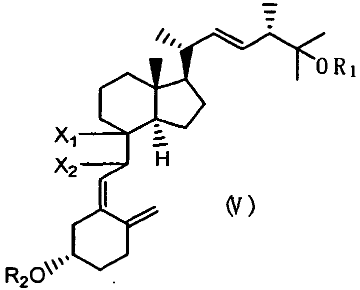 wherein in formula (V), both X₁ and X₂ denote hydroxyl groups or jointly form an epoxy group, and each of R₁ and R₂ independently denotes a hydrogen atom or a protective group for a hydroxyl group, characterized by comprising the step of oxidizing the vitamin D₂ derivative denoted by formula (IV) 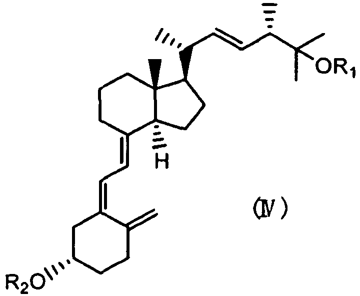 wherein in formula (IV), both R₁ and R₂ are defined as in formula (V).
[10] The preparing method in accordance with [9], wherein the oxidation of the vitamin D₂ derivative denoted by formula (IV) is conducted in solvent with an oxidizing agent.
[11] The preparing method in accordance with [9] or [10], wherein the protective groups for hydroxyl groups denoted by R₁ and R₂ are silyl groups, alkoxymethyl groups, aralkyloxymethyl groups, or acyl groups.
[12] The preparing method in accordance with [9] or [10], wherein the protective groups for hydroxyl groups denoted by R₁ and R₂ are trimethylsilyl groups, triethylsilyl groups, methoxymethyl groups, benzyloxymethyl groups, or acetyl groups.
[13] The preparing method in accordance with any one of [9] to [12], wherein the vitamin D₂ derivative denoted by formula (V) is the 7,8,25-trihydroxyvitamin D₂ denoted by formula (II) 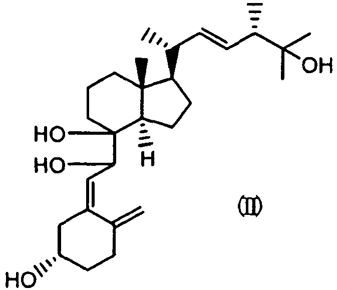 and the vitamin D₂ derivative denoted by formula (IV) is the 25-hydroxyvitamin D₂ denoted by formula (I) 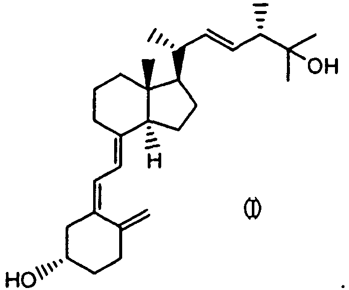
[14] A vitamin D₂ derivative denoted by formula (V) 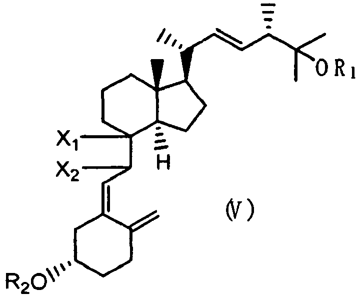 wherein in formula (V), both X₁ and X₂ denote hydroxyl groups or jointly form an epoxy group and each of R₁ and R₂ independently denotes a hydrogen atom or a protective group for a hydroxyl group.
[15] The derivative in accordance with [14], wherein the protective groups for hydroxyl groups denoted by R₁ and R₂ are silyl groups, alkoxymethyl groups, aralkyloxymethyl groups, or acyl groups.
[16] The derivative in accordance with [14], wherein the protective groups for hydroxyl groups denoted by R₁ and R₂ are trimethylsilyl groups, triethylsilyl groups, methoxymethyl groups, benzyloxymethyl groups, or acetyl groups.
[17] The derivative in accordance with [14], wherein R₁ and R₂ denote hydrogen atoms.
[18] The derivative in accordance with [14], wherein X₁ and X₂ both denote hydroxyl groups and R₁ and R₂ denote hydrogen atoms.
[19] A method for preparing an indene derivative denoted by formula (III) 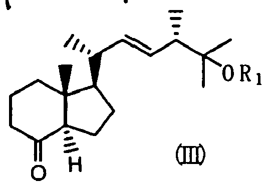 wherein in formula (III), R₁ denotes a hydrogen atom or a protective group for a hydroxyl group, characterized by comprising oxidizing a vitamin D₂ derivative denoted by formula (IV) 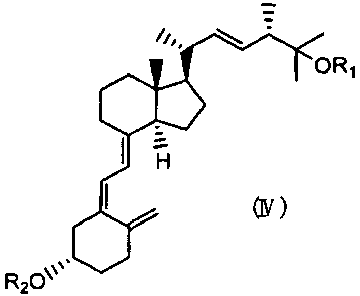 wherein in formula (IV), each of R₁ and R₂ independently denotes a hydrogen atom or a protective group for a hydroxyl group.
[20] The preparing method in accordance with [19], wherein the oxidation of the vitamin D₂ derivative denoted by formula (IV) is conducted in solvent with an oxidizing agent.
[21] The preparing method in accordance with [19] or [20], wherein the protective groups for the hydroxyl groups denoted by R₁ and R₂ are silyl groups, alkoxymethyl groups, aralkyloxymethyl groups, or acyl groups.
[22] The preparing method in accordance with [19] or [20], wherein the protective groups for the hydroxyl groups denoted by R₁ and R₂ are trimethylsilyl groups, triethylsilyl groups, methoxymethyl groups, benzyloxymethyl groups, or acetyl groups.

According to the preparing method of the present invention, which passes through the intermediate in the form of 7,8,25-trihydroxyvitamin D₂, or a derivative thereof, the indene derivative denoted by formula (III) is efficiently prepared from a starting compound in the form of 25-hydroxyvitamin D₂, or a derivative thereof, in a short preparing process.

### [Best Mode for Carrying Out the Invention]

The present invention covers the vitamin D₂ derivatives denoted by formula (V):

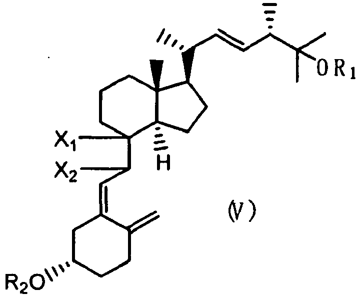

In formula (V), both X₁ and X₂ denote hydroxyl groups or jointly form an epoxy group. Each of R₁ and R₂ independently denotes a hydrogen atom or a protective group for a hydroxyl group. Examples of protective groups for hydroxyl groups are silyl groups, alkoxymethyl groups, aralkyloxymethyl groups, and acyl groups. More specific examples are trimethylsilyl groups, triethylsilyl groups, methoxymethyl groups, benzyloxymethyl groups, and acetyl groups.

### (A) Method for preparing the vitamin D₂ derivative denoted by formula (V)

The vitamin D₂ derivative denoted by formula (V) above can be prepared by oxidizing the vitamin D₂ derivative denoted by formula (IV)

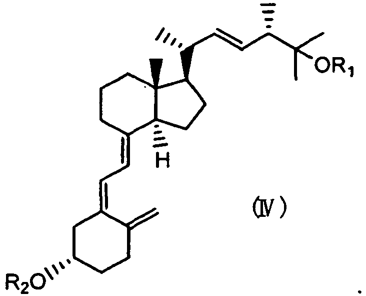

In formula (IV), R₁ and R₂ are defined as in formula (V). That is, each of R₁ and R₂ independently denotes a hydrogen atom or a protective group for a hydroxyl group. Examples of protective groups for hydroxyl groups are silyl groups, alkoxymethyl groups, aralkyloxymethyl groups, and acyl groups. More specific examples are trimethylsilyl groups, triethylsilyl groups, methoxymethyl groups, benzyloxymethyl groups, and acetyl groups. The vitamin D₂ derivative denoted by formula (IV) can be obtained by the following method, over the following pathway, or by the method described in the following literature.

The preparing method described in Example 4 of Japanese Patent No. 3,201,411 and the English language family member thereof, U.S. Patent 5,474,923 (title: Biological Production of Vitamin D) permits the obtaining of 25-hydroxyvitamin D₂ from vitamin D₂.

The hydroxyl protective group can be introduced by the method described in Protective Groups in Organic Synthesis, 3rd Ed. (T. W. Green, Wiley InterScience).

The vitamin D₂ derivative denoted by formula (V) can be obtained by oxidizing a starting compound in the form of the vitamin D₂ derivative denoted by formula (IV). The 7,8,25-trihydroxyvitamin D₂ denoted by formula (II), which is an implementation form of the vitamin D₂ derivative denoted by formula (V), can be similarly obtained by oxidation of a starting compound in the form of the 25-hydroxyvitamin D₂ denoted by formula (I). The oxidation can be done in a suitable solvent with an oxidizing agent. Examples of the oxidizing agent are permanganates (such as sodium and potassium salts), cetyltrimethylammonium permanganate, osmium tetraoxide, and m-chloroperbenzoic acid. It suffices to gradually add the oxidizing agent in a ratio of about 1 to 10 mols per mol of starting compound and stir. The oxidation from formula (IV) to formula (V) and the oxidation from formula (I) to formula (II) is desirably conducted using a permanganate (such as a sodium or potassium salt), cetyltrimethylammonium permanganate, or osmium tetraoxide. m-Choroperbenzoic acid is desirably employed in oxidation from formula (I) to formula (V) (in formula (V), both X₁ and X₂ denote jointly form an epoxy group.). The solvent is not specifically limited other than that it does not negatively affect the reaction. Examples of solvents suitable for use are hydrophilic organic solvents such as ethanol, acetonitrile, and acetone; mixtures thereof with water; and halomethane-based solvents such as dichloromethane and chloroform. The reaction time is from 5 minutes to 30 hours, and the reaction temperature is desirably from -75 to 80°C.

### (B) Method for preparing indene derivative (1)

The indene derivative denoted by formula (III) can be obtained by oxidizing the vitamin D₂ derivative denoted by formula (V), which serves as starting compound. Similarly, the indene derivative denoted by formula (III)' can be obtained by oxidizing the 7,8,25-trihydroxyvitamin D₂ denoted by formula (II) is an implementation form of the vitamin D₂ derivative denoted by formula (V).

The oxidation can be conducted, for example, in a suitable solvent with an oxidizing agent. Examples of oxidizing agents suitable for use are sodium periodate, iodobenzene acetate, [bis (trifluoroacetoxy)iodo]benzene, and lead tetraacetate. It suffices to gradually add the oxidizing agent in a ratio of about 1 to 10 mols per mol of starting compound and stir.

A periodate (such as sodium salt or potassium salt), orthoperiodate, lead tetraacetate, periodate (such as a sodium or potassium salt)/support, iodobenzene acetate, or [bis(trifluoroacetoxy)iodo]benzene can be employed in the oxidation from formula (V) to formula (III) and in the oxidation from formula (II) to formula (III)'. A periodate (such as a sodium or potassium salt), periodate (such as a sodium salt or potassium salt)/support, or iodobenzene acetate is desirably employed.

The solvent is not specifically limited other than that it dose not negatively affect the reaction. Examples of solvents suitable for use are methanol, ethanol, acetonitrile, acetone, dimethylether, halomethane-based solvents, benzene, and toluene; and mixtures thereof with water. The reaction time is 5 minutes to 30 hours and the reaction temperature is desirably -75 to 80°C.

The above oxidation can also be implemented using an oxidizing agent supported on a solid support as described in the above examples of the oxidizing agent. Examples of solid supports are silica gel, alumina, cerite, montmorillonite and the like. (C) below can be consulted for how to use solid supports. Use in combination with a supported oxidizing agent prevents the precipitation of starting material and product in the reaction system and accelerates the reaction. Compared to when conducted without a supported oxidizing agent, the reaction residue is cleaner, and post-processing and purification are simpler.

### (C) Method for preparing indene derivatives (2)

The indene derivative denoted by formula (III) can be obtained by oxidizing a starting compound in the form of the vitamin D₂ derivative denoted by formula (IV). Similarly, the indene derivative denoted by formula (III) can be obtained by oxidizing a starting material in the form of the 7,8,25-trihydroxyvitamin D₂ denoted by formula (II) that is an implementation form of the vitamin D₂ derivative denoted by formula (V).

The oxidation can be implemented using an oxidizing agent supported on a solid support. For example, periodates (such as sodium and potassium salts) and lead tetraacetate can be employed as the oxidizing agent, with permanganates being preferred. The solid support is not specifically limited other than that it dose not negatively affect the oxidizing agent, starting material, or solvent employed; the product; or the reaction. Examples of supports that can be employed in the reaction are silica gel (acid or neutral), alumina (acid, neutral, or alkaline), montmorillonite, and cerite. Preferably silica gel, alumina, cerite, montmorillonite and the like can be used. It suffices to gradually add in a ratio of about 1 to 10 mols per mol of starting compound and stir. Specifically, when employing potassium permanganate as the oxidizing agent, the weight ratio of potassium permanganate:support ranges from 1:1 to 1:10, desirably from 1:2 to 1:5, and preferably, from 1:2 to 1:4. The reagent can be added in a quantity (molar ratio) of from 1 to 30 equivalents, desirably 5 to 15 equivalents, relative to the starting material. The solvent is not specifically limited other than that it does not negatively affect the reaction, but an aqueous solvent is desirable. Examples are water-saturated ester solvents (such as methyl acetate, ethyl acetate, isopropyl acetate, butyl acetate and the like), and halogenated hydrocarbon solvents (such as dichloromethane, chloroform and the like). The reaction can be conducted at a temperature of from -75°C to the boiling point of the solvent, and is desirably conducted at room temperature (10 to 25°C). The reaction period can be from 0.5 hour to overnight, and is desirably from 1.5 hours to overnight (12 to 20 hours).

Once the reaction has ended, the target compound of the reaction can be recovered from the reaction mixture by the usual methods. For example, when insoluble material is present, suitable filtration can be conducted and then the solvent can be distilled off under reduced pressure. The reaction mixture also can be diluted with an organic solvent such as ethyl acetate, the mixture can be washed with water, the organic layer can be dried with magnesium sulfate anhydride, and the solvent can be distilled off to obtain the target compound. As needed, purification can be conducted by the usual methods, such as column chromatography, thin-layer chromatography, high-performance liquid chromatography, crystallization and the like.

### [Examples]

The present invention is described through examples below. However, the present invention is not limited to the forms given below.

### Example 1: Synthesis of 7,8,25-trihydroxyvitamin D₂

25-Hydroxyvitamin D₂ (101 mg, 0.24 mmol) was dissolved in ethanol (5 mL), an aqueous solution (2.5 mL) of potassium permanganate (77 mg, 0.48 mmol) was added dropwise with salt/ice cooling, and the mixture was stirred for 1.5 hours at -15 to 12°C and 5 minutes at 40°C. The reaction solution was centrifugally separated (3,000 rpm, 5 minutes). The supernatant was recovered and concentrated, yielding the crude 7,8,25-trihydroxyvitamin D₂ (116 mg) denoted below as a white amorphous product.

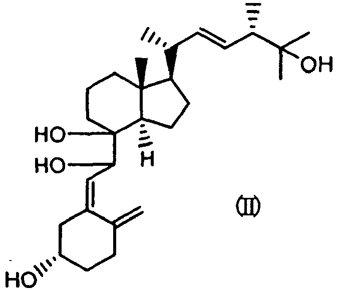

H-NMR (MeOD) δ*(delta)* (ppm): 5.51 (dd, 1H, J=2 and 10 Hz, H6), 5.34 (dd, 1H, J=8 and 15 Hz, H23), 5.23 (dd, 1H, J=8 and 15 Hz, H22), 5.00 (s, 1H, H19a), 4.99 (s, 1H, H19b), 4.90 (d, 1H, J=10 Hz, H7), 3.65 (m, 1H, H3), 2.54 (m, 1H, H4a), 2.43 (m, 1H, H1a), 2.13 to 2.11 (overlap, 1H, H4b), 2.12 to 2.00 (overlap, 4H, H1b, H2a, H20 and H24), 1.96 (m, 1H,H15a), 1.83 (overlap, 2H, H9a and H12a), 1.75 (m, 1H, H15b), 1.62 (m, 1H, H16a), 1.51 (dd, 1H, J=7 and 13 Hz, H 14), 1.47 to 1.44 (overlap, 2H, H2b and H11a), 1.30 (m, 1H, H11b), 1.21 to 1.13 (overlap, 3H, H12b, H16b and H17), 1.13 (overlap, 1H, H9b), 1.13 (s, 3H, H26), 1.09 (s, 3H, H27), 1.00 (d, 3H, J=7 Hz, H21), 0.99 (d, 3H, J=7 Hz, H28), 0.85 (s, 3H, H18)

¹³C-NMR (MeOD) δ*(delta)* (ppm): 147.42 (C10), 140.95 (C5), 138.37 (C22), 131.39 (C23), 126.71 (C6), 111.71 (C19), 76.29 (C8), 73.33 (C25), 71.46 (C3 or C7), 71.44 (C3 or C7), 61.06 (C14), 58.85 (C17), 51.12 (C13), 49.10 (C24), 47.66 (C4), 45.22 (C13), 41.57 (C12), 41.53 (C20), 39.12 (C9), 37.27 (C2), 34.52 (C1), 29.00 (C16), 28.28 (C26), 26.07 (C27), 22.89 (C15), 21.75 (C11), 21.04 (C21), 15.65 (C28), 13.66 (C18) ESI-MS: 469 [M+Na]⁺, 481 [M+Cl]⁻

### Example 2: Synthesis of indene derivative (III)'

The crude 7,8,25-trihydroxyvitamin D₂ (116 mg, 0.24 mmol) obtained in Example 1 was dissolved in methanol (3 mL). An aqueous solution (2.0 mL) of sodium periodate (100 mg, 0.47 mmol) was added with ice cooling. The reaction ended when the mixture had been stirred for 1 hour at 0°C. The reaction solution was concentrated, diluted with water (10 mL), and extracted with ethyl acetate (3 times with 10 mL). The organic layer was sequentially washed with 1 mol/L of hydrochloric acid (2 mL), saturated sodium bicarbonate aqueous solution (2 mL), and saturated sodium chloride aqueous solution (2 mL). The organic layer was dried with sodium sulfate, filtered, and concentrated. The residue was purified by silica gel chromatography (silica gel 60 N (spherical, neutral, 63 to 210 micrometers, Kanto Chemical Co.), 1.7 g, n-hexane/ethyl acetate = 3/1), yielding the indene derivative (III)' denoted below in the form of a white powder (32 mg, 45 % yield, two-steps).

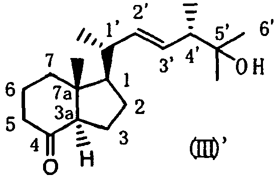

¹H-NMR (MeOD) *δ(delta)* (ppm): 5.38 (dd, 1H, J=8 and 15 Hz, H3'), 5.28 (dd, 1H, J=8 and 15 Hz, H2'), 2.61 (dd, 1H, J=7 and 11Hz, 3a-H), 2.31 (m, 1H, H5a), 2.18 (m, 1H, H5b), 2.12 to 2.05 (overlap, 3H, H7a, H1' and H4'), 2.05 to 2.01 (overlap, 1H, H6a), 1.92 (m, 1H, H6b), 1.75 (m, 1H, H2a), 1.68 (m, 1H, H3a), 1.64 (m, 1H, H7b), 1.55 (m, 1H, H1), 1.46 (m,1H, H3b), 1.32 (m, 1H, H2b), 1.12 (s, 3H, H6'), 1.11 (s, 3H, 5'-Me), 1.07 (d, 3H, J=7 Hz, H1'-Me), 0.99 (d, 3H, J=7 Hz, H4'-Me), 0.65 (s, 3H, 7a-Me)

¹³C-NMR (MeOD) *δ(delta)* (ppm) : 214.95 (C4), 137.84 (C2'), 131.94 (C3'), 73.32 (C5'), 62.91 (C3a), 57.76 (C1), 51.12 (C7a), 49.10 (C4'), 41.78 (C5), 41.28 (C 1'), 39.90 (C7), 28.85 (C2), 28.27 (C6'), 26.18 (5'-Me), 25.23 (C6), 21.35 (1'-Me), 20.07 (C3), 15.66 (4'-Me), 13.09 (7a-Me)
ESI-MS: 315 [M+Na] ⁺, 327 [M+Cl]⁻

### Example 3: Synthesis of 7,8-epoxy-25-hydroxyvitamin D₂

25-Hydroxyvitamin D₂ (100 mg, 0.24 mmol) was dissolved in methylene chloride (2 mL), m-chloroperbenzoic acid (62.7 mg, 0.36 mmol) was added with ice cooling, and the mixture was stirred for 1 hour with ice cooling. The reaction solution was diluted with 30 mL of ethyl acetate, and sequentially washed twice with 15 mL of 5 percent sodium bicarbonate aqueous solution and twice with 15 mL of saturated sodium chloride aqueous solution. The organic layer was then dried with sodium sulfate anhydride. The solvent was removed and the residue was separated and purified by preparative TLC (Merck, 5744, Hex./EtOAc = 1/2), yielding 7,8-epoxy-25-hydroxyvitamin D₂ (706 mg, 68 % yield).

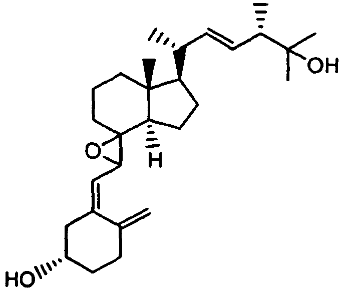

¹H-NMR (CDCl₃) δ *(delta)* (ppm) : 5.37 to 5.21(m, 2H, H22,H23), 5.22(d, 1H, J=9.3, H6), 5.02(s, 1H,H19a), 4.94(s, 1H, H19b), 4.00(m, 1H, H3), 3.86(d, 1H, J=9.3 Hz, H7), 2.60(dd, 1H, J=13.1 and 3.7 Hz, H4a), 2.48(m, 1H, H1a), 2.31(dd, 1H, *J=13.1* and 7.1 Hz, H4b), 2.17(m, 1H, H1b), 2.12 to 2.05(overlap, 2H, H20, H24), 1.94(overlap, 1H, H2a), 1.73 (overlap, 1H, H2b), 1.96 to 1.05 (overlap, 12H, H9, H11, H12, H 14, H15, H16, H17), 1.16(s, 3H, H26), 1.13(s, 3H, H27), 1.00(d, 3H, J=6.6 Hz, H21), 0.99(d, 3H, J=6.9 Hz, H28), 0.70(s, 3H, H18)
ESI-MS : 451 [M+Na]⁺, 427[M]⁻

### Example 4: Synthesis of indene derivative (III)'

The 7,8-epoxy-25-hydroxyvitamin D₂ (700 mg) obtained in Example 3 was dissolved in THF (2 mL). Orthoperiodate (55 mg, 0.24 mmol) was added with ice cooling, after which 0.5 mL of water was added dropwise. The mixture was stirred for 1 hour with ice cooling and orthoperiodate (55 mg, 0.24 mmol) was added. The mixture was stirred for another 2 hours with ice cooling, orthoperiodate (55 mg, 0.24 mmol) was added, the mixture was stirred for 1 hour, and the reaction was ended. The reaction solution was diluted with 30 mL of ethyl acetate and sequentially washed twice with 15 mL of 5 percent sodium bicarbonate aqueous solution and twice with 15 mL of saturated sodium chloride aqueous solution. The organic layer was dried with sodium sulfate anhydride. The solvent was removed and the residue was separated and purified by preparative TLC (Merck, 5744, Hex./EtOAc = 2/1), yielding indene derivative (III)' (5.3 mg, 7.5 % yield, two steps).

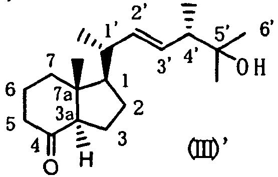

### Example 5: Synthesis of 7,8-dihydroxy-3-triethylsilyl-25-triethylsiloxyvitamin D₂

3-Triethylsilyl-25-triethylsiloxyvitamin D₂ (50 mg, 0.078 mmol) was dissolved in ethanol (5 mL) and an aqueous solution (0.85 mL) of potassium permanganate (34 mg, 0.21 mmol) was added dropwise with ice cooling. The mixture was reacted for 2.5 hours at room temperature and the reaction solution was filtered through cerite. The filtrate was concentrated, and separated and purified by preparative TLC (Merck 5744, Hex./EtOAc = 20/1, 4/1), yielding the target compound (3.8 mg, 3.8 %). 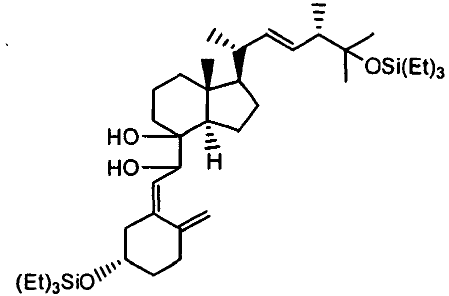 ¹H-NMR (CDCl₃) δ*(delta)* (ppm) : 5.51 (near dd, 1H, J=10 Hz, H6), 5.30 (dd, 1H, J=8 and 15 Hz, H23), 5.18 (dd, 1H, J=8 and 15 Hz, H22), 4.98 (s, 1H, H19a), 4.91 (d, 1H, J=10 Hz, H7), 4.88 (s, 1H, H19b), 3.69 (m, 1H, H3), 2.35-1.00 (m, 20H), 1.15 (s, 3H, H26), 1.10 (s, 3H, H27), 0.97 (m, 24H, Me and TES-Me), 0.88 (s, 3H, H18), 0.62 (s, 12H, TES-CH2)
ESI-MS: 711 [M+Cl]⁻

### Example 6: Synthesis of 7,8-hydroxy-3-methoxymethyl-25-methoxymethyloxyvitamin D₂

3-Methoxymethyl-25-methoxymethyloxyvitamin D₂ (50 mg, 0.1 mmol) was dissolved in ethanol (5 mL) and an aqueous solution (1.1 mL) of potassium permanganate (44 mg, 0.28 mmol) was added dropwise with ice cooling. The mixture was reacted for 2.5 hours at room temperature, after which the reaction solution was filtered through cerite. The filtrate was concentrated, and separated and purified by preparative TLC (Merck 5744, Hex./EtOAc = 5/1), yielding the target compound shown below (9.1 mg, 17.0 %). 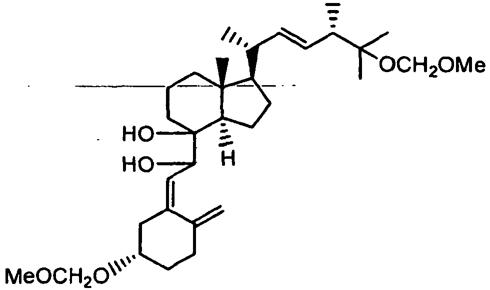 ¹H-NMR (CDCl₃) δ*(delta)* (ppm) : 5.56 (near dd, 1H, J=10 Hz, H6), 5.31 (dd, 1H, J=8 and 15 Hz, H23), 5.21 (dd, 1H, J=8 and 15 Hz, H22), 5.00 (s, I H, H 19a), 4.93 (d, I H, J=10 Hz, H7), 4.91 (s, 1H, H 19b), 4.71 and 4.68 (each s, 4H, MOM-CH2), 3.63 (m, 1H, H3), 3.39 and 3.37 (each s, 6H, MOM-Me), 2.42-1.00 (m, 20H), 1.13 (s, 3H, Me), 1.10 (s, 3H, Me), 0.98 (comp m, 6H, Me), 0.89 (s, 3H, H18)
ESI-MS: 557 [M+Na] ⁺, 569 [M+Cl]⁻

### Example 7: Synthesis of 3-benzyloxymethyl-25-benzyloxymethyloxy-7,8-dihydroxyvitamin D₂

3-Benzyloxymethyl-25-benzyloxymethyloxyvitamin D₂ (50 mg, 0.076 mmol) was dissolved in ethanol (5 mL) and an aqueous solution (0.85 mL) of potassium permanganate (34 mg, 0.21 mmol) was added dropwise with ice cooling. The mixture was reacted for 2.5 hours at room temperature, after which the reaction solution was filtered through cerite. The filtrate was concentrated, and separated and purified by preparative TLC (Merck 5744, Hex./EtOAc = 5/1 x 2), yielding the target compound shown below (5.6 mg, 10.7 %). 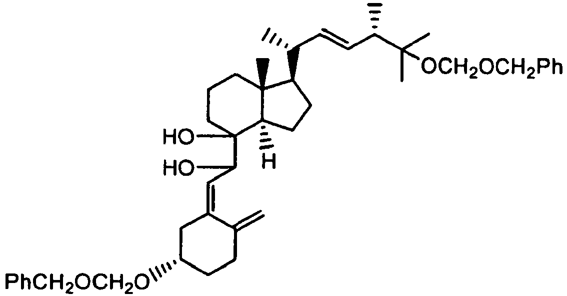 H-NMR (CDCl₃) δ*(delta)* (ppm) : 7.30 (m, 10H, BOM-Ph), 5.56 (near dd, 1H, J=10 Hz, H6), 5.33 (dd, 1H, J=8 and 16 Hz, H23), 5.22 (dd, 1H, J=8 and 16 Hz, H22), 5.01 (s, 1H, H19a), 4.91 (near D₂H, H19b and H7), 4.85, 4.82 and 4.63 (each s, 8, BOM-CH2), 3.70 (m, 1H, H3), 2.42-1.0 (m, 20H), 1.25 and 1.18 (each s, 3H x 2, Me), 0.98 (comp m, 6H, Me), 0.80 (s, 3H, H18)
ESI-MS: 709 [M+Na] ⁺, 686 [M]⁻

### Example 8: Synthesis of 3-acetyl-25-acetoxy-7,8-dihydroxyvitamin D₂

3-Acetyl-25-acetoxyvitamin D₂ (36 mg, 0.072 mmol) was dissolved in ethanol (3.6 mL) and an aqueous solution (0.8 mL) of potassium permanganate (32 mg, 0.2 mmol) was added dropwise with ice cooling. The mixture was reacted for 1 hour at room temperature and the reaction solution was filtered through cerite. The filtrate was concentrated, and separated and purified by preparative TLC (Merck 5744, Hex./EtOAc = 3/2), yielding the target compound (5.6 mg, 14.7 %) shown below. 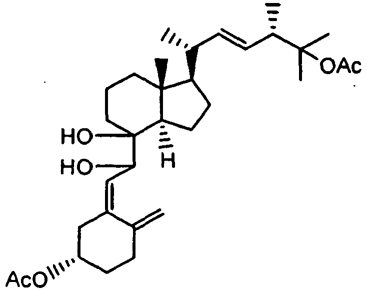 ¹H-NMR (CDCl₃) δ*(delta)* (ppm) : 5.58 (near dd, 1H, J=10 Hz, H6), 5.25 (m, 2H, H22 and H23), 5.03 (s, 1H, H19a), 4.96 (s, 1H, H19b), 4.90 (dd, J=5 and 10 Hz, 1H, H7), 4.871 (m, 1H, H3), 2.60-1.0 (m, 20H), 2.04 (s, 3H, Ac), 1.96 (s, 3H, Ac), 1.39 (s, 3H, Me), 1.37 (s, 3H, Me), 0.96 (comp m, 6H, Me), 0.81 (s, 3H, H18)
ESI-MS: 553 [M+Na]⁺, 565 [M+Cl]⁻

### Example 9: Synthesis of 5'-triethylsiloxyindene derivative

3-Triethylsilyl-25-triethylsiloxyvitamin D₂ (50 mg, 0.078 mmol) was dissolved in ethanol (5 mL) and an aqueous solution (0.85 mL) of potassium permanganate (34 mg, 0.21 mmol) was added dropwise with ice cooling. The mixture was reacted for 6.5 hours at room temperature, the reaction solution was filtered through cerite, and the cerite employed in filtration was washed with ethanol (10 mL). The washed ethanol and the filtrate were combined and the mixture was concentrated, yielding a 7,8-diol product. Methanol (1.5 mL) was added. A solution comprised of sodium periodate (50 mg, 0.23 mmol) dissolved in purified water (0.5 mL) and sodium periodate (116 mg, 0.078 mmol) supported on a silica gel were added with ice cooling. The reaction was returned to room temperature and continued for 4 hours. The reaction solution was filtered through cerite and the cerite employed in filtration was washed with ethyl acetate (10 mL). The filtrates were combined and the mixture was concentrated. The mixture was separated and purified by preparative TLC (Merck 5744, Hex./EtOAc = 4/1), yielding a trace quantity of the target compound.

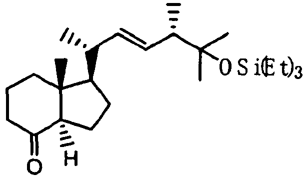

¹H-NMR (CDCl₃) δ*(delta)* (ppm) : 5.33 (dd, 1H, J=8 and 15 Hz, H3'), 5.19 (dd, 1H, J=8 and 15 Hz, H2'), 2.45 (dd, 1H, J=7 and 11Hz, 3a-H), 2.30 to 2.18 (overlap, 2H, H5a and H5b), 2.11 to 2.09 (m, 1H, H7a), 2.08 to 1.99 (overlap, 3H, H1', H4' and H6a), 1.91 (m, 1H, H6b), 1.75 to 1.66 (overlap, 2H, H2a and H3a), 1.59 (m, 1H, H7b), 1.51 to 1.44 (overlap, 2H, H1 and H3b), 1.32 (m, 1H, H2b), 1.15 (s, 3H, H6'), 1.10 (s, 3H, H5'-Me), 1.04 (d, 3H, J=7 Hz, H1'-Me), 0.95 (t, 9H, J=8 Hz, H5'-TES-CH3), 0.95 (overlap, 3H, H4'-Me), 0.65 (s, 3H, 7a-Me), 0.57 (q, 6H, J=8 Hz, H5'-TES-CH2)
ESI-MS: 429 [M+Na] ⁺

### Example 10: Synthesis of 5'-methoxymethyloxyindene derivative

3-Methoxymethyl-25-methoxymethyloxyvitamin D₂ (50 mg, 0.1 mmol) was dissolved in ethanol (5 mL) and an aqueous solution (1.1 mL) of potassium permanganate (44 mg, 0.28 mmol) was added dropwise with ice cooling. The mixture was reacted for 7.5 hours at room temperature, the reaction solution was filtered through cerite, and the cerite employed in filtration was washed with ethanol (10 mL). The washed ethanol and the filtrate were combined and the mixture was concentrated, yielding 7,8-diol product. Methanol (1.5 mL) was added. A solution comprised of sodium periodate (64 mg, 0.3 mmol) dissolved in purified water (0.5 mL) and sodium periodate (149 mg, 0.1 mmol) supported on a silica gel were added with ice cooling. The reaction was warmed up to room temperature and continued for 3.5 hours. The reaction solution was filtered through cerite and the cerite employed in the filtration was washed with ethyl acetate (10 mL). The washed ethyl acetate and the filtrate were combined and the mixture was concentrated. The mixture was separated and purified by preparative TLC (Merck 5744, Hex./EtOAc = 4/1), yielding 0.4 mg (1.2 %) of the target compound.

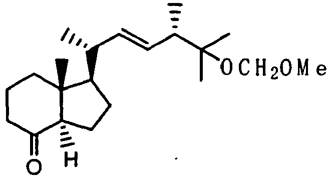

¹H-NMR (CDCl₃) *δ(delta)* (ppm) : 5.34 (dd, 1H, J=8 and 15 Hz, H3'), 5.24 (dd, 1 H, J=8 and 15 Hz, H2'), 4.71 (s, 2H, -MOM-CH2), 3.36 (s, 3H, -MOM-CH3), 2.45 (dd, 1H, J=7 and 11 Hz, 3a-H), 2.30 to 2.18 (overlap, 3H, H4', H5a and H5b), 2.10 to 1.98 (overlap, 3H, H1', H6a and H7a), 1.89 (m, 1H, H6b), 1.77 to 1.66 (overlap, 2H, H2a and H3a), 1.59 (m, 1H, H7b), 1.52 to 1.45 (overlap, 2H, H 1 and H3b), 1.30 (m, 1H, H2b), 1.16 (s, 3H, H6'), 1.13 (s, 3H, H5'-Me), 1.04 (d, 3H, J=7 Hz, H1'-Me), 0.99 (d, 3H, J=7 Hz, H4'-Me), 0.65 (s, 3H, 7a-Me)
ESI-MS: 359 [M+Na]⁺

### Example 11: Synthesis of 5'-benzyloxymethyloxyindene derivative

3-Benzyloxymethyl-25-benzyloxymethoxyvitamin D₂ (50 mg, 0.076 mmol) was dissolved in ethanol (5 mL) and an aqueous solution (0.85 mL) of potassium permanganate (34 mg, 0.21 mmol) was added dropwise with ice cooling. The mixture was reacted for 7.5 hours at room temperature, the reaction solution was filtered through cerite, and the cerite employed in filtration was washed with ethanol (10 mL). The washed ethanol and the filtrate were combined and the mixture was concentrated, yielding a 7,8-diol product. Methanol (1.5 mL) was added. A solution comprised of sodium periodate (49 mg, 0.227 mmol) dissolved in purified water (0.5 mL) and sodium periodate (113 mg, 0.076 mmol) supported on a silica gel were added with ice cooling. The reaction was returned to room temperature and continued for 4 hours. The reaction solution was filtered through cerite and the cerite employed in the filtration was washed with ethyl acetate (10 mL). The washed ethyl acetate and the filtrate were combined and the mixture was concentrated. The mixture was separated and purified by preparative TLC (Merck 5744, Hex./EtOAc = 4/1), yielding 4.4 mg (14.0 %) of the target compound.

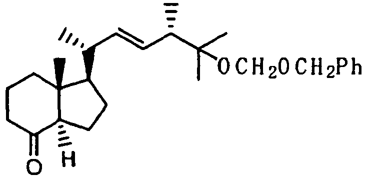

¹H-NMR (CDCl₃) *δ(delta)* (ppm) : 7.34 to 7.26 (m, 5H, Ph), 5.36 (dd, 1H, J=8 and 15 Hz, H3'), 5.25 (dd, 1H, J=8 and 15 Hz, H2'), 4.72 (s, 2H, -BOM-CH2), 4.63 (s, 2H, -BOM-CH2), 2.44 (dd, 1H, J=8 and 11 Hz, 3a-H), 2.31 to 2.18 (overlap, 3H, H4', H5a and H5b), 2.11 to 1.98 (overlap, 3H, H1', H6a and H7a), 1.91 (m, 1H, H6b), 1.76 to 1.66 (overlap, 2H, H2a and H3a), 1.59 (m, 1H, H7b), 1.51 to 1.45 (overlap, 2H, H1 and H3b), 1.32 (m, 1H, H2b), 1.20 (s, 3H, H6'), 1.17 (s, 3H, H5'-Me), 1.04 (d, 3H, J=7 Hz, H1'-Me), 1.00 (d, 3H, J=7 Hz, H4'-Me), 0.65 (s, 3H, 7a-Me)
ESI-MS: 435 [M+Na] ⁺

### Example 12: Synthesis of 5'-acetyloxyindene derivative

3-Acetyl-25-acetoxyvitamin D₂ (50 mg, 0.1 mmol) was dissolved in ethanol (5 mL) and an aqueous solution (1.1 mL) of potassium permanganate (44 mg, 0.28 mmol) was added dropwise with ice cooling. The mixture was reacted for 7.5 hours at room temperature, the reaction solution was filtered through cerite, and the cerite employed in the filtration was washed with ethanol (10 mL). The washed ethanol and the filtrate were combined and the mixture was concentrated, yielding a 7,8-diol product. Methanol (1.5 mL) was added. A solution comprised of sodium periodate (64 mg, 0.3 mmol) dissolved in purified water (0.5 mL) and sodium periodate (149 mg, 0.1 mmol) supported on a silica gel were added with ice cooling. The reaction was returned to room temperature and continued for 4 hours. The reaction solution was filtered through cerite and the cerite employed in the filtration was washed with ethyl acetate (10 mL). The washed ethyl acetate and the filtrate were combined and the mixture was concentrated. The mixture was separated and purified by preparative TLC (Merck 5744, Hex./EtOAc = 4/1), yielding 4.4 mg (10.2 %) of the target compound.

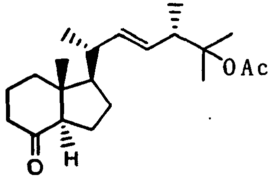

H-NMR (CDCl₃) δ*(delta)* (ppm) : 5.25 (near dD₂H, H2' and H3'), 2.78-1.20 (comp m, 14H) 1.96 (s, 3H, Ac), 1.39 (s, 3H, Me), 1.37 (s, 3H, Me), 1.04 (d, 3H, J=7 Hz, Me), 0.97 (d, 3H, J=7 Hz, Me), 0.65 (s, 3H, 7a-Me),
ESI-MS: 357 [M+Na] ⁺

### Example 13: Synthesis of indene derivative (III)' employing supported permanganate

A solution comprised of potassium permanganate (1.58 g, 0.01 mol) dissolved in purified water (100 mL) was added at room temperature to cerite (5 g) that had been washed with hydrochloric acid. The mixture was intimately stirred for 30 minutes after which the water was removed under reduced pressure, yielding supported potassium permanganate as a dark purple solid (6.60 g, about 1.5 mmol/g). A 340 mg (0.51 mmol) quantity of this solid was weighed out and added to water-saturated ethyl acetate (0.2 mL). A solution comprised of 25-hydroxyvitamin D₂ (20 mg, 0.048 mmol) dissolved in water-saturated ethyl acetate (0.2 mL) was added dropwise at room temperature. The mixture was reacted for 4 hours at room temperature and the solid was removed by filtration. The filtrate was concentrated, and separated and purified by preparative TLC (Hex./EtOAc = 2/1), yielding the targeted product in the form of white crystals (4.6 mg, 32.8 %).

By the same method as above, the following reaction was implemented using silica gel (acid), alumina (acid), alumina (basic), or montmorillonite K10 instead of cerite washed with hydrochloric acid. The results are given below together with those for the reactions employing cerite washed with hydrochloric acid (washed cerite).

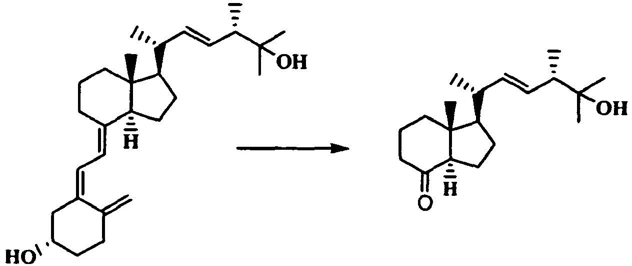

| | Reagent | Solvent | Yield (%) |
|---|---|---|---|
| 1 | KMnO₄/silica gel (acid) | EtOAc (sat. H₂O) | 22 |
| 2 | KMnO₄/alumina (acid) | EtOAc (sat. H₂O) | 29 |
| 3 | KMnO₄/alumina (basic) | EtOAc (sat. H₂O) | 31 |
| 4 | KMnO₄/washed cerite | EtOAc (sat. H₂O) | 33 |
| 5 | KMnO₄/montmorillonite K 10 | CH2C 12 (sat. H₂O) | 27 |

### Example 14: Synthesis of indene derivative (III)' using supported periodate

A solution comprised of sodium periodate (8 g, 37.4 mol) dissolved in purified water (15.6 mL) was added at room temperature to silica gel (Merck, 1.07734, 5009) (31 g). The mixture was intimately stirred for 50 minutes at 80°C and the water was removed under reduced pressure, yielding supported sodium periodate as a white solid (55.7 g, about 0.67 mmol/g).

The crude 7,8,25-trihydroxyvitamin D₂ (20.5 mg) obtained in Example 1 was dissolved in methanol (0.6 mL) and an aqueous solution (200 microliters) of sodium periodate (26.7 mg, 0.124 mmol) and supported sodium periodate (73.5 mg, 0.049 mmol) were added with ice cooling. The mixture was stirred for 16 hours at room temperature, at which time the reaction ended. The reaction solution was filtered through cerite and concentrated. The residue was separated and purified by preparative TLC (Merck 5744, n-hexane/ethyl acetate = 8/1), yielding the indene derivative (III)' (5.4 mg, 37 % yield) denoted below in the form of a white powder.

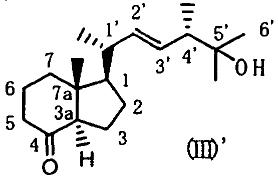

### Example 15: Synthesis of indene derivative (III)'

25-Hydroxyvitamin D₂ (1 g, 2.42 mmol) was dissolved in ethanol (100 mL) and an aqueous solution (25 mL) of potassium permanganate (956 mg, 6.05 mmol) was added dropwise at -40°C. Upon completion of the dropwise addition, the cooling device was turned off and the temperature was raised from -40°C to -25°C over a period of 40 minutes. The mixture was stirred for 20 minutes at room temperature. The reaction solution was filtered through cerite. The cerite was washed with ethanol (20 mL x 2), and the ethanol and the filtrate were combined. After filtration through filter paper (Advantech, No. 2 filter paper), the filtrate was concentrated, yielding crude 7,8,25-trihydroxyvitamin D₂. This was dissolved in methanol (50 mL). An aqueous solution (15 mL) of sodium periodate (3.64 g, 17 mmol) was added dropwise with ice cooling. Following the dropwise addition, the mixture was stirred for 2 hours at room temperature. The reaction solution was filtered through cerite, after which the organic solvent was removed under reduced pressure. Ethyl acetate (20 mL x 2) was added to the remaining aqueous layer, which was extracted. The organic layer obtained was washed with purified water and saturated sodium chloride aqueous solution, and then dried with sodium sulfate. The solution was filtered and concentrated. The residue obtained was purified by silica gel column chromatography (silica gel 60 N (spherical, neutral, 63 to 210 micrometers, Kanto Chemical Co.) 100 mL, n-hexane/ethyl acetate = 9/1 to 7/1 to 5/1), yielding the indene derivative (III)' denoted below in the form of a white powder (365.5 g, 52 % yield). The structure was confirmed by comparison with the data obtained in Example 1.

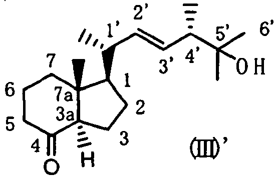

### Example 16: Synthesis of indene derivative (III)'

25-Hydroxyvitamin D₂ (41 mg, 0.1 mmol) was dissolved in dichloromethane (0.6 mL) and a dichloromethane solution (1.8 mL) of cetyltrimethylammonium permanganate (120 mg, 0.3 mmol) was added dropwise with ice cooling. The mixture was then stirred overnight at room temperature. Dichloromethane (0.5 mL) was added to the reaction solution. Subsequently, a dichloromethane solution (2 mL) of iodobenzene acetate (64 mg) was added at room temperature. The mixture was stirred for 2 hours and concentrated. Acetonitrile (4 mL) was added to the residue and the mixture was vigorously stirred. The reaction solution was filtered through cerite and the cerite was washed with acetonitrile (2 mL), and the acetonitrile and the filtrate were combined. The residue was separated and purified by preparative TLC (Merck silica gel 60 F254 1.05744, n-hexane/ethyl acetate = 1/1), yielding the indene derivative (III)' denoted below in the form of a white powder (8 mg, 27 % yield). The structure was confirmed by comparison with the data obtained in Example 1.

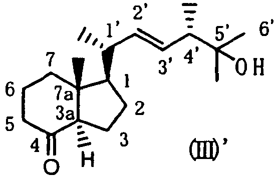

### Reference example: Preparation of cetyltrimethylammonium permanganate

It was prepared with reference to Synthesis 1984, 431.

Potassium permanganate (1.58 g, 10 mmol) was dissolved in purified water (50 mL). A solution of cetyltrimethylammonium bromide (4.01 g, 11 mmol) in purified water (50 mL) was added. The mixture was stirred for 40 minutes, after which the purple precipitate that appeared was filtered out with a Kiriyama funnel (40 φ *phi*, filter paper no. 4). The precipitate obtained was washed with purified water until the filtrate became transparent. This was dried at 35°C under reduced pressure, yielding the targeted cetyltrimethylammonium permanganate (3.21 g, 79.5 %).

The reaction pathways of the respective example are indicated below.

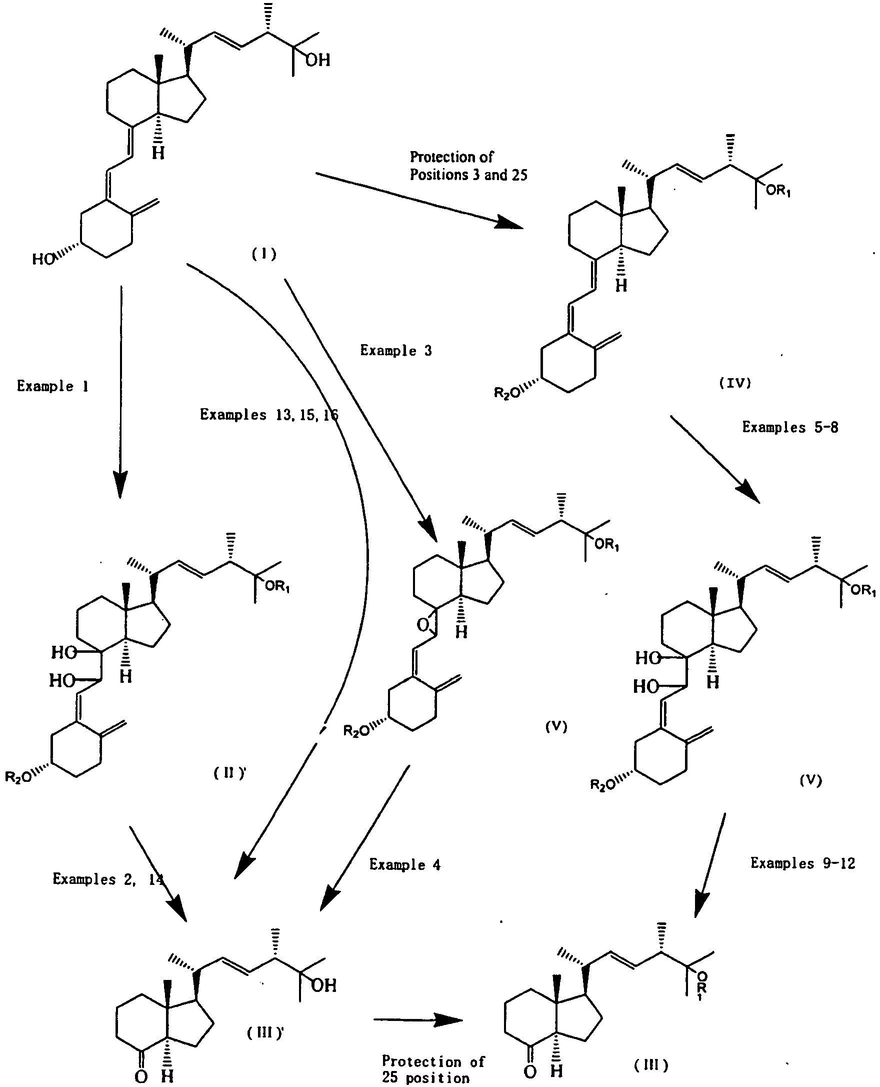

## Claims

1. A method of preparing an indene derivative denoted by formula (III) 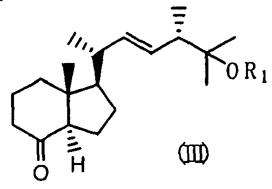 wherein in formula (III), R₁ denotes a hydrogen atom or a protective group for a hydroxyl group, **characterized by** comprising oxidizing a vitamin D₂ derivative denoted by formula (V) 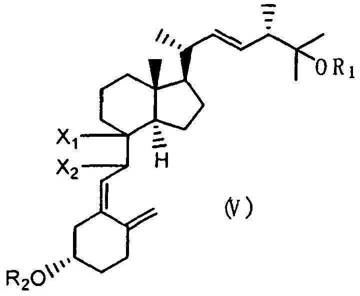 wherein in formula (V), both X₁ and X₂ denote hydroxyl groups or jointly form an epoxy group, and each of R₁ and R₂ independently denotes a hydrogen atom or a protective group for a hydroxyl group.

2. The preparing method in accordance with claim 1, wherein the protective groups for hydroxyl groups denoted by R₁ and R₂ are silyl groups, alkoxymethyl groups, aralkyloxymethyl groups, or acyl groups.

3. The preparing method in accordance with claim 1, wherein the vitamin D₂ derivative denoted by formula (V) is 7,8,25-trihydroxyvitamin D₂ denoted by formula (II)

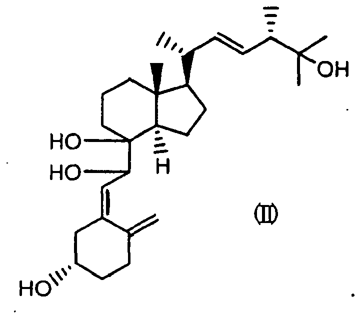

4. The preparing method in accordance with any one of claims 1 to 3, wherein the vitamin D₂ derivative denoted by formula (V) is prepared by oxidizing the vitamin D₂ derivative denoted by formula (IV) 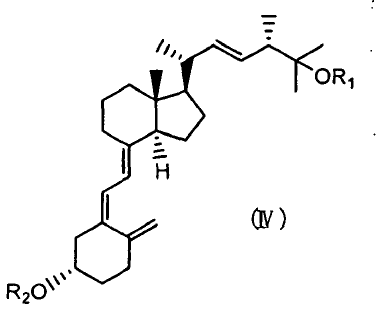 wherein in formula (IV), both R₁ and R₂ are defined as in formula (V).

5. The preparing method in accordance with claim 3, wherein the 7,8,25-trihydroxyvitamin D₂ denoted by formula (II) is prepared by oxidizing the 25-hydroxyvitamin D₂ denoted by formula (I)

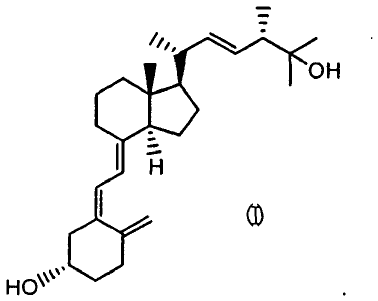

6. A method of preparing a vitamin D₂ derivative denoted by formula (V) 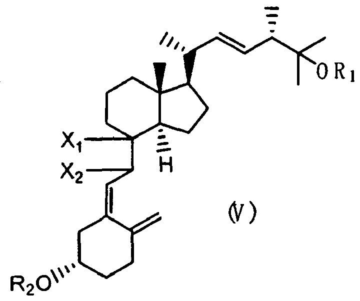 wherein in formula (V), both X₁ and X₂ denote hydroxyl groups or jointly form an epoxy group, and each of R₁ and R₂ independently denotes a hydrogen atom or a protective group for a hydroxyl group, **characterized by** comprising the step of oxidizing the vitamin D₂ derivative denoted by formula (IV) 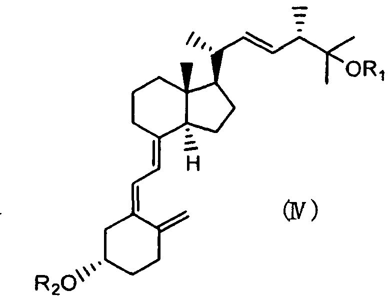 wherein in formula (IV), both R₁ and R₂ are defined as in formula (V).

7. The preparing method in accordance with claim 6, wherein the protective groups for hydroxyl groups denoted by R₁ and R₂ are silyl groups, alkoxymethyl groups, aralkyloxymethyl groups, or acyl groups.

8. The preparing method in accordance with claim 6 or 7, wherein the vitamin D₂ derivative denoted by formula (V) is the 7,8,25-trihydroxyvitamin D₂ denoted by formula (II) 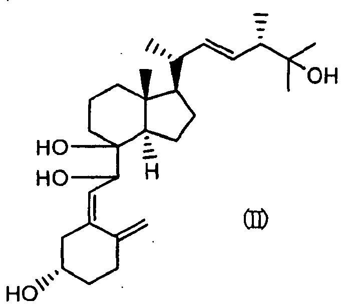 and the vitamin D₂ derivative denoted by formula (IV) is the 25-hydroxyvitamin D₂ denoted by formula (I)

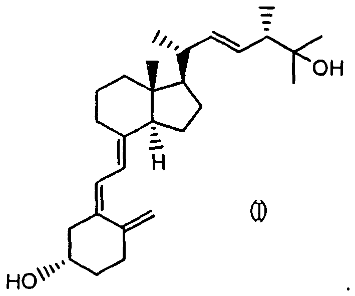

9. A vitamin D₂ derivative denoted by formula (V) 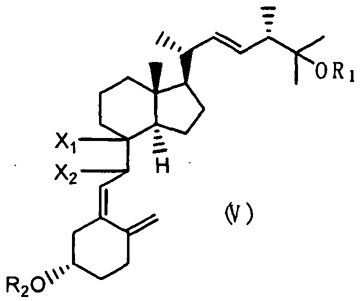 wherein in formula (V), both X₁ and X₂ denote hydroxyl groups or jointly form an epoxy group and each of R₁ and R₂ independently denotes a hydrogen atom or a protective group for a hydroxyl group.

10. The derivative in accordance with claim 9, wherein the protective groups for hydroxyl groups denoted by R₁ and R₂ are silyl groups, alkoxymethyl groups, aralkyloxymethyl groups, or acyl groups.

11. The derivative in accordance with claim 9, wherein R₁ and R₂ denote hydrogen atoms.

12. The derivative in accordance with claim 9, wherein X₁ and X₂ both denote hydroxyl groups and R₁ and R₂ denote hydrogen atoms.

13. A method of preparing an indene derivative denoted by formula (III) 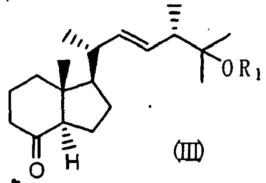 wherein in formula (III), R₁ denotes a hydrogen atom or a protective group for a hydroxyl group, **characterized by** comprising oxidizing the vitamin D₂ derivative denoted by formula (IV) 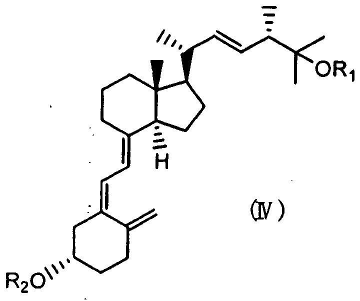 wherein in formula (IV), each of R₁ and R₂ independently denotes a hydrogen atom or a protective group for a hydroxyl group.

14. The preparing method in accordance with claim 13, wherein the protective groups for the hydroxyl groups denoted by R₁ and R₂ are silyl groups, alkoxymethyl groups, aralkyloxymethyl groups, or acyl groups.

## Patentansprüche

1. Verfahren zur Herstellung eines Indenderivats, wie in der Formel (III) dargestellt 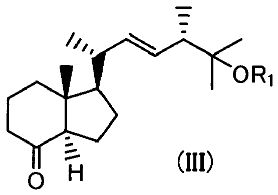 wobei in Formel (III) R₁ ein Wasserstoffatom oder eine Schutzgruppe für eine Hydroxylgruppe bezeichnet, **dadurch gekennzeichnet, dass** es umfasst:
Oxidieren eines Derivats von Vitamin D₂, wie in der Formel (V) dargestellt 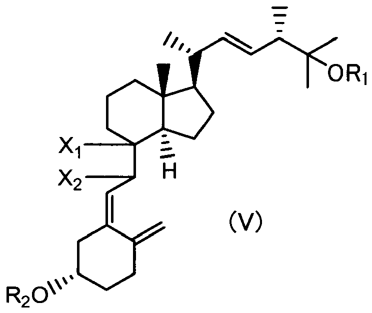 wobei in Formel (V) sowohl X₁ wie X₂ Hydroxylgruppen bezeichnen oder gemeinsam eine Epoxidgruppe bilden und R₁ und R₂ jeweils unabhängig ein Wasserstoffatom oder eine Schutzgruppe für eine Hydroxylgruppe bezeichnen.

2. Herstellungsverfahren nach Anspruch 1, wobei die Schutzgruppen für Hydroxylgruppen, die mit R₁ und R₂ bezeichnet sind, Silylgruppen, Alkoxymethylgruppen, Aralkyloxymethylgruppen oder Acylgruppen sind.

3. Herstellungsverfahren nach Anspruch 1, wobei das in Formel (V) dargestellte Derivat von Vitamin D₂ ein in Formel (II) dargestelltes 7,8,25-Trihydroxy-Vitamin D₂ ist

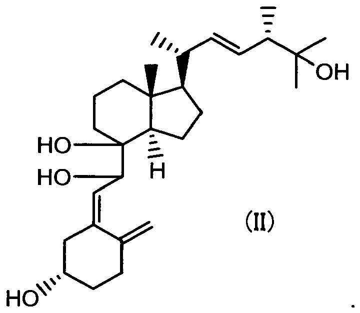

4. Herstellungsverfahren nach einem der Ansprüche 1 bis 3, wobei das in Formel (V) dargestellte Derivat von Vitamin D₂ durch Oxidieren des in Formel (IV) dargestellten Derivats von Vitamin D₂ gebildet wird 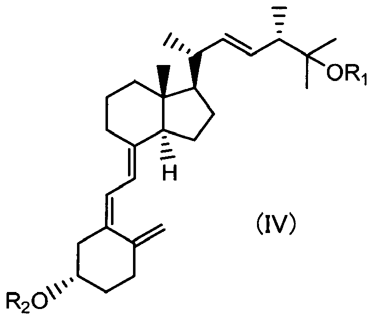 wobei in Formel (IV) sowohl R₁ und R₂ wie in Formel (V) definiert sind.

5. Herstellungsverfahren nach Anspruch 3, wobei das in Formel (II) dargestellte 7,8,25-Trihydroxy-Vitamin D₂ durch Oxidieren des in Formel (I) dargestellten 25-Hydroxy-Vitamin D₂ gebildet wird

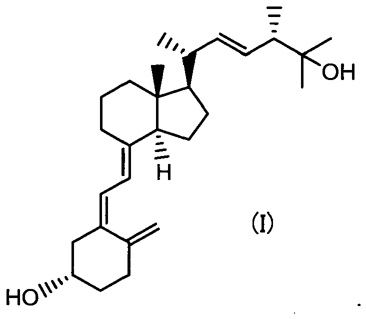

6. Verfahren zur Herstellung eines Derivats von Vitamin D₂, wie in der Formel (V) dargestellt 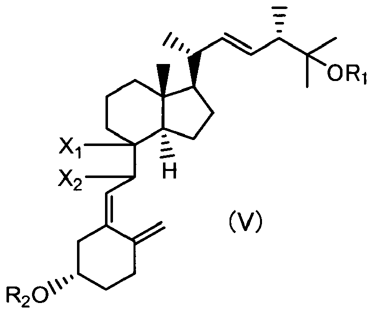 wobei in Formel (V) sowohl X₁ wie X₂ Hydroxylgruppen bezeichnen oder gemeinsam eine Epoxidgruppe bilden und R₁ und R₂ jeweils unabhängig ein Wasserstoffatom oder eine Schutzgruppe für eine Hydroxylgruppe bezeichnen, **dadurch gekennzeichnet, dass** es den Schritt umfasst:
Oxidieren des in der Formel (IV) dargestellten Derivats von Vitamin D₂ 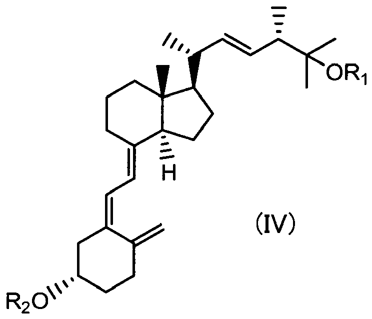 wobei in Formel (IV) sowohl R₁ und R₂ wie in Formel (V) definiert sind.

7. Herstellungsverfahren nach Anspruch 6, wobei die Schutzgruppen für Hydroxylgruppen, die mit R₁ und R₂ bezeichnet sind, Silylgruppen, Alkoxymethylgruppen, Aralkyloxymethylgruppen oder Acylgruppen sind.

8. Herstellungsverfahren nach Anspruch 6 oder 7, wobei das in Formel (V) dargestellte Derivat von Vitamin D₂ das in Formel (II) dargestelltes 7,8,25-Trihydroxy-Vitamin D₂ ist 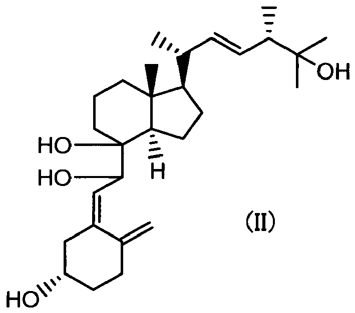 und das in Formel (IV) dargestellte Derivat von Vitamin D₂ das in Formel (I) dargestellte 25-Hydroxy-Vitamin D₂ ist

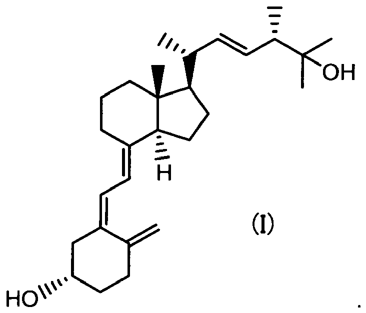

9. Derivat von Vitamin D₂, wie in Formel (V) dargestellt 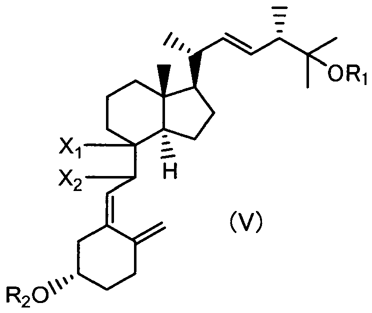 wobei in Formel (V) sowohl X₁ wie X₂ Hydroxylgruppen bezeichnen oder gemeinsam eine Epoxidgruppe bilden und R₁ und R₂ jeweils unabhängig ein Wasserstoffatom oder eine Schutzgruppe für eine Hydroxylgruppe bezeichnen.

10. Derivat nach Anspruch 9, wobei die Schutzgruppen für Hydroxylgruppen, die mit R₁ und R₂ bezeichnet sind, Silylgruppen, Alkoxymethylgruppen, Aralkyloxymethylgruppen oder Acylgruppen sind.

11. Derivat nach Anspruch 9, wobei R₁ und R₂ Wasserstoffatome bezeichnen.

12. Derivat nach Anspruch 9, wobei sowohl X₁ wie X₂ Hydroxylgruppen bezeichnen und R₁ wie R₂ Wasserstoffatome bezeichnen.

13. Verfahren zur Herstellung eines Indenderivats, wie in der Formel (III) dargestellt 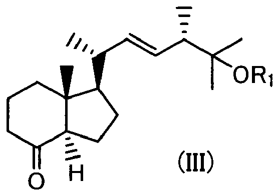 wobei in Formel (III) R₁ ein Wasserstoffatom oder eine Schutzgruppe für eine Hydroxylgruppe bezeichnet, **dadurch gekennzeichnet, dass** es umfasst:
Oxidieren des in der Formel (IV) dargestellten Derivats von Vitamin D₂ 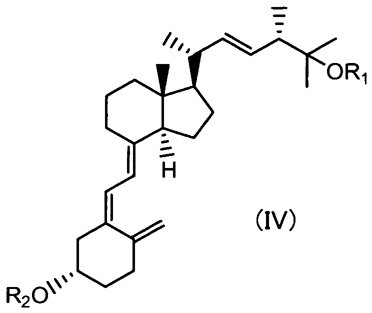 wobei in Formel (IV) R₁ und R₂ jeweils unabhängig ein Wasserstoffatom oder eine Schutzgruppe für eine Hydroxylgruppe bezeichnen.

14. Herstellungsverfahren nach Anspruch 13, wobei die Schutzgruppen für Hydroxylgruppen, die mit R₁ und R₂ bezeichnet sind, Silylgruppen, Alkoxymethylgruppen, Aralkyloxymethylgruppen oder Acylgruppen sind.

## Revendications

1. Méthode de préparation d'un dérivé d'indène désigné par la formule (III) 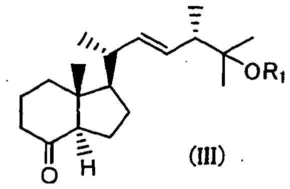 dans laquelle, dans la formule (III), R₁ désigne un atome d'hydrogène ou un groupement protecteur pour un groupement hydroxyle, **caractérisée en ce qu'**elle comprend l'oxydation d'un dérivé de vitamine D₂ désigné par la formule (V) 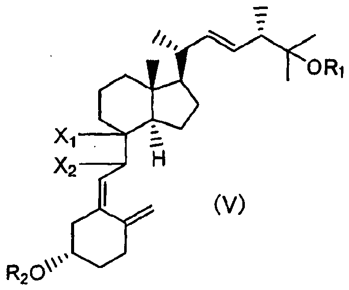 dans laquelle, dans la formule (V), X₁ ainsi que X₂ désignent des groupements hydroxyle ou forment conjointement un groupement époxy, et chacun parmi R₁ et R₂ désigne indépendamment un atome d'hydrogène ou un groupement protecteur pour un groupement hydroxyle.

2. Méthode de préparation selon la revendication 1, dans laquelle les groupements protecteurs pour les groupements hydroxyle désignés par R₁ et R₂ sont des groupements silyle, des groupements alcoxyméthyle, des groupements aralkyloxyméthyle ou des groupements acyle.

3. Méthode de préparation selon la revendication 1, dans laquelle le dérivé de vitamine D₂ désigné par la formule (V) est la 7,8,25-trihydroxy-vitamine D₂ désignée par la formule (II)

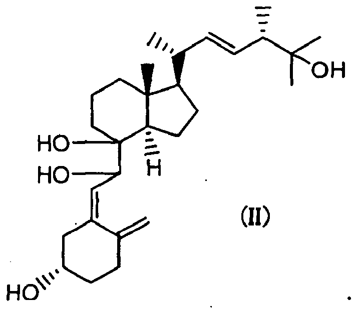

4. Méthode de préparation selon l'une quelconque des revendications 1 à 3, dans laquelle le dérivé de vitamine D₂ désigné par la formule (V) est préparé par l'oxydation du dérivé de vitamine D₂ désigné par la formule (IV) 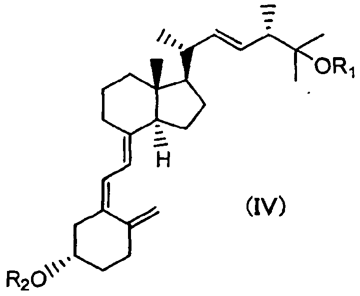 dans laquelle, dans la formule (IV), R₁ ainsi que R₂ sont tels que définis dans la formule (V).

5. Méthode de préparation selon la revendication 3, dans laquelle la 7,8,25-trihydroxy-vitamine D₂ désignée par la formule (II) est préparée par l'oxydation de la 25-hydroxy-vitamine D₂ désignée par la formule (I)

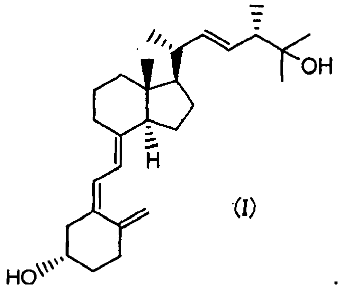

6. Méthode de préparation d'un dérivé de vitamine D₂ désigné par la formule (V) 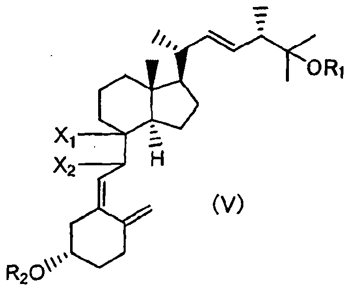 dans laquelle, dans la formule (V), X₁ ainsi que X₂ désignent des groupements hydroxyle ou forment conjointement un groupement époxy, et chacun parmi R₁ et R₂ désigne indépendamment un atome d'hydrogène ou un groupement protecteur pour un groupement hydroxyle, **caractérisée en ce qu'**elle comprend l'étape d'oxydation du dérivé de vitamine D₂ désigné par la formule (IV) 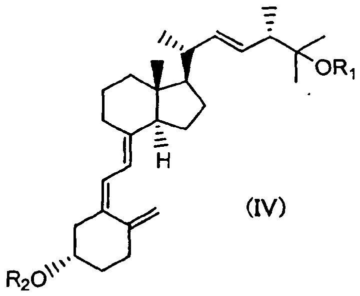 dans laquelle, dans la formule (IV), R₁ ainsi que R₂ sont tels que définis dans la formule (V).

7. Méthode de préparation selon la revendication 6, dans laquelle les groupements protecteurs pour les groupements hydroxyle désignés par R₁ et R₂ sont des groupements silyle, des groupements alcoxyméthyle, des groupements aralkyloxyméthyle ou des groupements acyle.

8. Méthode de préparation selon la revendication 6 ou 7, dans laquelle le dérivé de vitamine D₂ désigné par la formule (V) est la 7,8,25-trihydroxy-vitamine D₂ désignée par la formule (II) 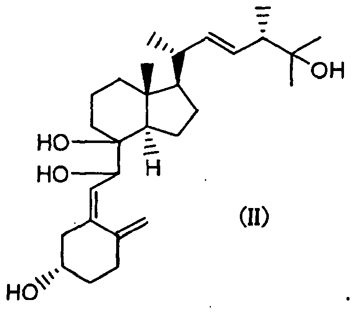 et le dérivé de vitamine D₂ désigné par la formule (IV) est la 25-hydroxy-vitamine D₂ désignée par la formule (I)

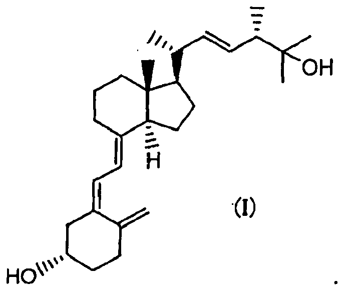

9. Dérivé de vitamine D₂ désigné par la formule (V) 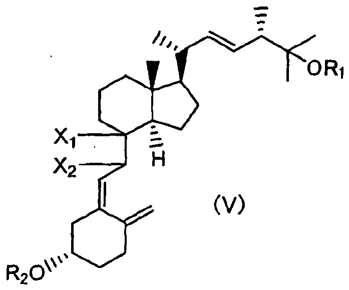 dans laquelle, dans la formule (V), X₁ ainsi que X₂ désignent des groupements hydroxyle ou forment conjointement un groupement époxy, et chacun parmi R₁ et R₂ désigne indépendamment un atome d'hydrogène ou un groupement protecteur pour un groupement hydroxyle.

10. Dérivé selon la revendication 9, dans lequel les groupements protecteurs pour les groupements hydroxyle désignés par R₁ et R₂ sont des groupements silyle, des groupements alcoxyméthyle, des groupements aralkyloxyméthyle ou des groupements acyle.

11. Dérivé selon la revendication 9, dans lequel R₁ et R₂ désignent des atomes d'hydrogène.

12. Dérivé selon la revendication 9, dans lequel X₁ ainsi que X₂ désignent des groupements hydroxyle et R₁ et R₂ désignent des atomes d'hydrogène.

13. Méthode de préparation d'un dérivé d'indène désigné par la formule (III) 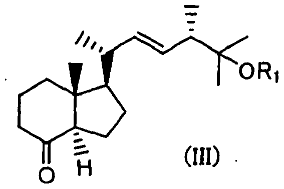 dans laquelle, dans la formule (III), Rᵢ désigne un atome d'hydrogène ou un groupement protecteur pour un groupement hydroxyle, **caractérisée en ce qu'**elle comprend l'oxydation du dérivé de vitamine D₂ désigné par la formule (IV) 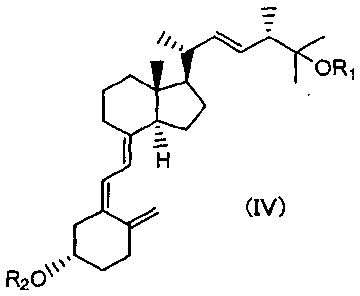 dans laquelle, dans la formule (IV), chacun parmi R₁ et R₂ désigne indépendamment un atome d'hydrogène ou un groupement protecteur pour un groupement hydroxyle.

14. Méthode de préparation selon la revendication 13, dans lequel les groupements protecteurs pour les groupements hydroxyle désignés par R₁ et R₂ sont des groupements silyle, des groupements alcoxyméthyle, des groupements aralkyloxyméthyle ou des groupements acyle.
